# EUROPEAN PATENT APPLICATION

(11) **EP 1 169 969 A1**
(43) Date of publication of application: **09.01.2002**
(21) Application number: 01115833.4
(22) Date of filing: 28.06.2001
(51) Int. Cl.: A61B 17/12

(54) **Pedicle occlusion device**

(30) Priority: 05.07.2000 US 610318
(71) Applicant: Medtronic AVE, Inc., Santa Rosa, California 95403 (US)
(72) Inventor: Williams, Michael S., Santa Rosa, CA 95409 (US)
(74) Representative: Hughes, Andrea Michelle

(57) **Abstract**

A device (10) for occluding fluid flow through a lumen, especially a small diameter blood vessel, is provided comprising a delivery configuration and a treatment configuration. Fluid flow through the lumen transforms the device from the delivery configuration to the treatment configuration. The device may comprise at least one filamentary strand (16), and may further comprise a body portion (12) attached to the at least on filamentary strand which may be rendered radiopaque.

## Description

The present invention relates to a device for permanent occlusion of a body lumen. More specifically, the invention relates to an effective occlusion device which has an ultra low-profile delivery configuration for site specific delivery within a body lumen such as within the intravascular system, particularly within small diameter vessels such as are present in the cerebrovascular tree. Further, the device readily transforms from the delivery configuration to a treatment configuration.

There are a number of conditions where the indicated treatment includes the permanent occlusion of a vessel or lumen within the human body. Some vessels for which occlusion is desired are of very small diameter and/or are located within and/or beyond tortuous regions of the vasculature.

One example of such a condition is an arteriovenous malformation. Arteriovenous malformations (AVMs)are an anomaly consisting of a tangle of vessels having different wall thicknesses and diameters and having abnormal communication between the arterial and venous system. AVMs are the most common intracranial vascular malformation.

Surgical removal of an AVM is invasive, and in some situations may pose insurmountable risk or difficulty, rendering endovascular therapy the preferable mode of treatment. The desired objective of endovascular therapy in such situations may be to eliminate blood supply from pedicles, feeders, or other blood supplying vessels to the anomaly, or to eliminate communication between the arterial and venous system.

Other examples of conditions for which occlusion of a blood vessel is desired include an inoperable tumor, or a tumor which must be treated preoperatively. In such situations, occlusion of one or more small diameter feeder vessels, or pedicles, may be desired. Blood supply to the tumor is thereby eliminated, shrinking, and potentially eliminating, the tumor.

Examples of other desired objectives of endovascular therapy in various conditions include: controlling internal bleeding; limiting bleeding during surgery or after trauma; and relieving vessel wall pressure in proximity to an aneurysm. Examples of non-vascular applications include therapeutic ureteric occlusion; fallopial occlusion; pulmonary occlusion; and septal defects occlusion.

Several approaches and devices have been employed to produce blockage or occlusion of vessels. Such approaches include the use of particulate embolic agents, embolic fluids, adhesives, detachable balloons, coils or coiled wire, and occlusion devices formed from an anchor member and an occlusive membrane or material, or some combination thereof. There are limitations, however, associated with each of these approaches, especially within the clinical context of the microvasculature.

A disadvantage of particulate embolic agents, or thrombosis-producing particles, is inaccurate placement. Such particles are typically released and then carried by blood flow to a point where the vessel diameter is equal to or less than the diameter of the particle. Occlusion of feeder vessels to an AVM is desirable at their entry into the mass.
Loss of control over the point of occlusion may also be undesirable in treating a tumor. Further, temporary occlusion with recanalization of the vessel after occlusion can result.

Adhesives include liquid compounds such as cyanoacrylates, which, when injected into a vessel, polymerize upon contact with blood to solidify within the lumen. There is very little control over the placement of the adhesive due to the cure rate of the adhesive within the bloodstream. Further, there is the risk of misdirection of the adhesive into a parent artery or other non-targeted vessel.

Disadvantages associated with balloon occlusion devices arise from their somewhat unpredictable and hazardous nature. Due to the stiffness of their delivery system, they cannot be advanced to more tortuous, distal regions, and therefore, are limited to larger vessels. Balloon occlusion devices also present the risk of premature detachment from their delivery systems. Furthermore, such a balloon can deflate or, on the other hand, rupture the vessel in which it is placed.

Coils used to occlude vessels have the disadvantage of the lack of control over or ability to be positioned accurately. Further, they are prone to migration once ejected out of the delivery catheter into the vessel. Additionally, coils often fail to produce complete occlusion of the target vessel and often result in the recanalization of the treatment site. And coils may not be suitable for use in smaller diameter vessels.

A stent with a flexible closure member attached thereto has also been posited as an endovascular means for occluding flow through a vessel. Stents are medical implant devices designed and conventionally employed for the treatment of chronic restenosis or other intravascular narrowing. Stents are typically delivered to an affected area in a vessel and then expanded to maintain the passageway patent. Thus, stents are designed to perform a function which is antithetic to intravascular occlusion.

The flexible closure member, attached to the stent, physically blocks a vessel passageway to effect occlusion. As the flexible or elastomeric member stretches, however, it will cause an increase in the recoil of the stent. Therefore, the stent would have to be oversized to prevent migration, increasing the risk of injury or dissection of the vessel. Moreover, due to the stress imposed by the stretchable material of the stent device, the device can disengage from the vessel wall after a period of time. Further, such a device may not be suitable for smaller diameter vessels.

Other devices have been proposed for occlusion of a body lumen. For example, the devices disclosed in U.S. Patent No. 5,209,799 are particularly effective in achieving permanent, total occlusion of a blood vessel.
However, such a device may not be specifically designed for treatment of very small diameter vessels.

Accordingly, there exists a need for an alternative intravascular occlusion device, particularly for occluding intracranial or other very small diameter vessels. More specifically, there is a need for one which effects rapid and total occlusion in a single device, and can be accurately positioned within small diameter vessels. And finally, there exists a need for an occlusion device which is capable of being advanced into the severe tortuosity such as that characterized by the cerebrovascular tree.

The invention herein is directed to an improved device which has an advantageous delivery configuration such that it can be accurately positioned within tortuous, small diameter vessels, and an advantageous treatment configuration such that it accomplishes permanent occlusion, with a single device. Further, the invention herein readily transforms from the delivery configuration to the treatment configuration, preferably taking advantage of blood flow to achieve the transformation. The improved device is of particular use for occlusion of small diameter feeder vessels or pedicles, and is capable of effecting rapid and total occlusion with little or no chronic revascularization. And finally, the invention herein is relatively easy to manufacture.

The device according to the present invention is a device for occluding fluid flow through a lumen, said device comprising: a delivery configuration and a treatment configuration. The device may further comprise a body portion attached to the tail-like structure. The body portion may be comprised of a polymer or metal, and may be molded or injection molded in conjunction with the tail-like portion, or the tail-like portion may be dipped into a polymeric matrix, such that the strands or fibers are encapsulated by the body portion. The body portion may further be comprised of substances which confer radiopacity of the device, and/or a therapeutic agent or agents.

The preferred device comprises a highly streamlined delivery configuration in which the tail-like structure extends in a substantially linear fashion. Such configuration is low-profile, highly flexible, low mass and low volume for delivery and particularly advantageous for delivery into small diameter vasculature. Such a streamlined configuration enables the device to be delivered via a microcatheter, potentially well within tortuous vasculature. The device may be delivered simultaneously with additional embolic or other therapeutic agents.

The device further comprises a treatment configuration in which, preferably, the tail-like structure is reconfigured from the streamlined, linear configuration into a ball-like structure.

The preferred embodiment takes advantage of blood flow to achieve ball-like treatment configuration. More specifically, the device is transformed from its delivery configuration into its treatment configuration by the flow of fluid through the lumen.

The occlusive properties of the device are attributed to mechanical, rheological and thrombogenic means. When in the treatment configuration, the device advantageously comprises a high surface area for effective occlusion. The ball-like structure mechanically occludes the vessel, disrupting flow, and further presenting a material which induces a thrombogenic response. These multiple mechanisms ensure occlusive efficacy.

The filamentary strands, fibers, or fiber bundles are preferably formed of materials which induce a thrombogenic response. Specifically, the filamentary strands are formed from materials selected from the group consisting of silk, cotton, polyethylene, polyethylene terephthalate, wool, collagen yarn, cellulose and the like. The strands or fibers can additionally be coated or impregnated with additional thrombogenic materials. Examples of thrombogenic materials include but are not limited to collagen, gelatin, thrombin, trypsin, peptides, protamine or polylysine.

The filamentary strands may also comprise other therapeutic agents including, but not limited to agents for inhibiting tumor growth, radioactive isotopes, angiogenic growth factors, cell cycle inhibitors, or other gene therapy agents. Additionally, the filamentary strands may comprise materials to enhance radiopacity. Such materials include, but are not limited to, Barium sulfate, Bismuth Trioxide, Platinum, Iridium, Gold or Tantalum or alloys of the aforementioned.

The device, while in its streamlined delivery configuration, is placed within very small vessels via a conventional microcatheter. Once accurately positioned, the device may be ejected from the microcatheter via either fluid, or a pusher wire, or both.

In the event the device is ejected via fluid, the fluid may also comprise a therapeutic or diagnostic agent. Examples of therapeutic agents include but are not limited to thrombogenic agents, therapies for inhibiting tumor growth, angiogenic growth factors, cell cycle inhibitors, and others. Examples of diagnostic agents include but are not limited to contrast medium, and agents for enhancement of radiographic visualization. Alternatively any mechanical, thermal, or electromechanical detachment and/or retrieval means known to one skilled in the art may be used to detach and deliver the device. Controlled detachment mechanisms such as those mentioned above may allow for retrieval of the device after placement. Prior to detachment such mechanisms allow for physician discretion and selection of precise deployment parameters and location.

Other modes of delivery may include retaining the occlusion device with a degradable binding agent which breaks down upon contact with the blood or after exposure to a given pH such as 7.4. After delivery, the device transforms from its substantially linear delivery configuration to its treatment configuration. More specifically, the light weight, fibrous tail portion of the device is carried by blood flow toward the more stationary body portion to form a ball-like structure with the body portion. Such configuration mechanically blocks blood flow and further creates a thrombogenic environment within the vessel, to permanently occlude the vessel.

Placement of the device may be enhanced by blood flow in a situation in which the treatment site is otherwise inaccessible. In such a situation, the body portion will be permitted to travel a limited distance and will eventually anchor the device. Once anchored, the device will transform from the delivery configuration to the treatment configuration, as described above.

Preferred embodiments will now be described, by way of example only, with reference to the drawings.
**FIG. 1** illustrates a perspective view of an embodiment of the invention in its delivery configuration.
**FIG. 2** illustrates a perspective view of an alternative embodiment of the invention in its delivery configuration.
**FIG. 3a** illustrates a perspective view of yet another alternative embodiment of the invention in its delivery configuration.
**FIG. 3b** illustrates a perspective view of the embodiment of FIG. 3a in its treatment configuration.
**FIG. 4** illustrates a perspective view of an embodiment of the invention within a microcatheter in a vessel, proximate an arteriovenous malformation.
**FIG. 5** illustrates the device according to one embodiment of the invention immediately following ejection of the device from the microcatheter.
**FIG. 6** illustrates an embodiment of the invention in its treatment configuration within the vessel.
**FIG. 7** illustrates an alternative embodiment of the invention.

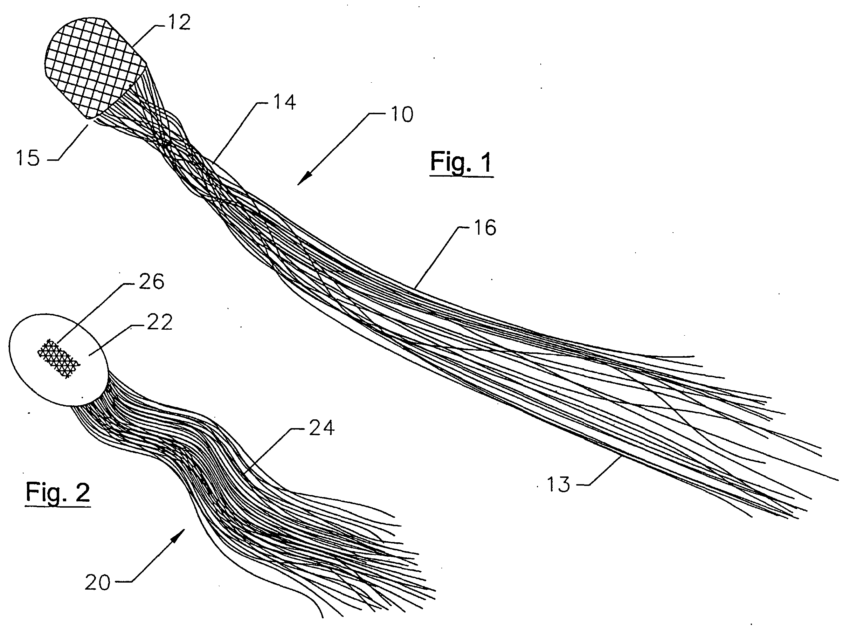

FIG. 1 illustrates an occlusion device 10 according to the present invention, said device comprising body portion 12 and tail portion 14. Tail portion 14 is comprised of a plurality of filamentary strands 16. Tail portion 14 has a first end 13 and a second end 15. Body portion 12 is formed at second end 15 of tail portion 14, and may be comprised of a molded polymer or a metal braid or mesh. Where body portion 12 is formed of a polymer, the device can be formed by dipping filamentary strands into a polymeric matrix.

FIG. 2 illustrates an alternative embodiment of the invention in its delivery configuration. The device 20 of said embodiment comprises body portion 22 and tail portion 24. Said embodiment further comprises radiopaque marker 26 on or in body portion 22. Radiopaque marker 26 may be Gold, Tantalum, Platinum, Iridium, any alloy thereof or any other substances conferring radiopacity. Body portion 22 may alternatively comprise Barium Sulfate, Bismuth Trioxide, or other substance conferring radiopacity.

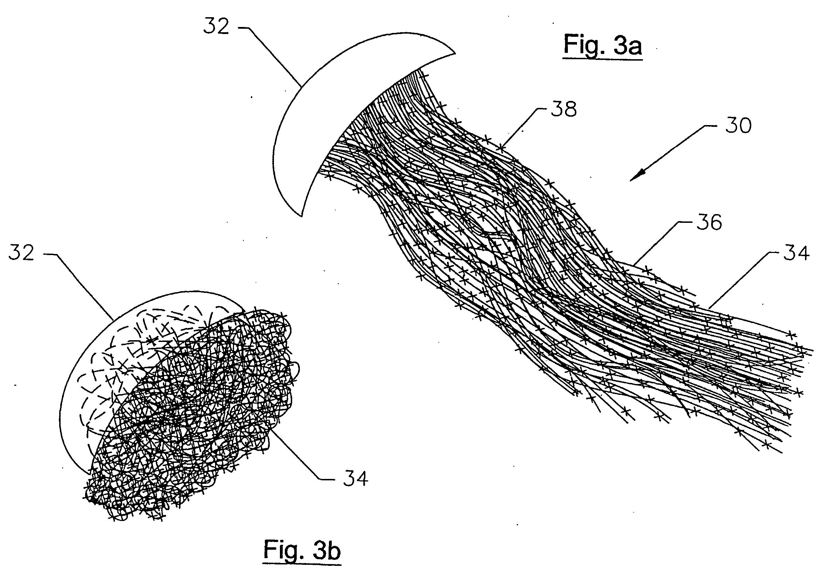

FIG. 3a depicts another alternative embodiment of the occlusion device 30 in its delivery configuration comprising body portion 32 and tail portion 34. Body portion 32 may comprise an elastomeric or other flexible material, such that body portion 32 can more flexibly conform to the interior of a lumen. Filamentary strands 36 of tail portion 34 comprise thrombogenic substance 38. Figure 3b depicts the embodiment of Figure 3a in its treatment configuration. Tail portion 34 is now configured in an amorphous structure in close proximity to body portion 32.

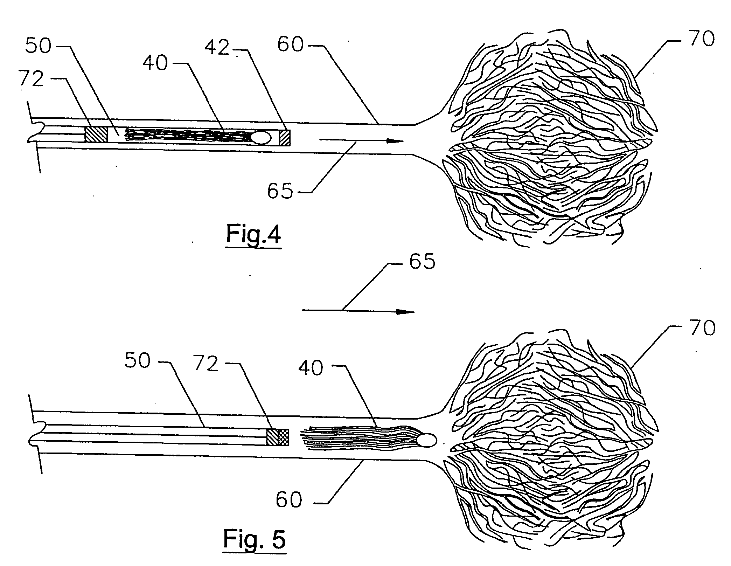

Turning now to FIG. 4, device 40 is depicted within microcatheter 50, which is within pedicle 60. Pedicle 60 is a feeder vessel to arteriovenous malformation 70. Microcatheter 50 comprises radiopaque marker 42 for improved visualization of catheter 50 within pedicle 60. Microcatheter 50 is advanced as near as possible to the nidus of arteriovenous malformation 70. The direction of blood flow is depicted by arrow 65. Once accurately positioned within the vessel, the device may be ejected from the microcatheter via fluid (not pictured), pusher wire 72, or both. Alternatively any known mechanical, thermal or electromechanical detachment means may be used to detach and deliver the device.

FIG. 5 depicts device 40 in its delivery configuration following ejection from microcatheter 50, proximate arteriovenous malformation 70. Following ejection from microcatheter 50, depending upon the diameter of pedicle 60, occlusion device 40 may be transported by blood flow in direction 65. Soon after ejection from microcatheter 50, device 40 will transform from its delivery configuration to its treatment configuration. Device 40 will no longer travel through pedicle 60, but rather will come to rest within the ostium of pedicle 60. Blood flow in direction 65 will then augment the reconfiguration of the device from the delivery configuration to the treatment configuration of device 40.

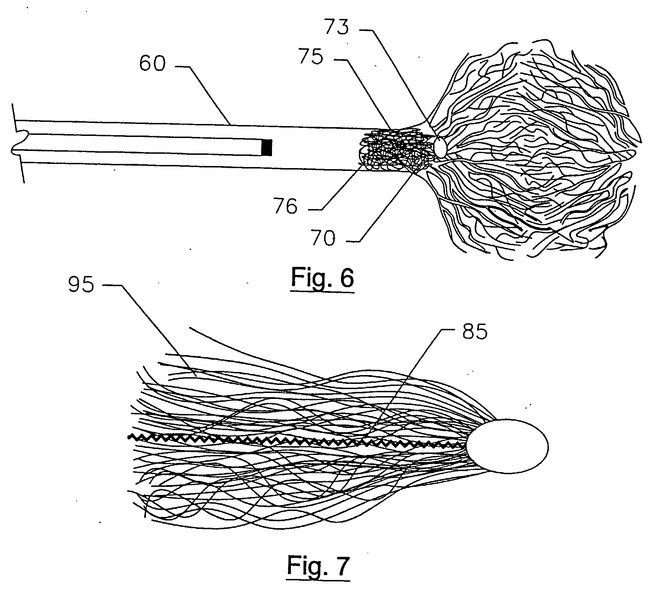

FIG. 6 depicts device 70 in its treatment configuration. Tail portion 76 and body portion 73 now define amorphous ball-like structure 75. Thrombus formation will initiate immediately, and device 70 combined with such thrombus will permanently occlude pedicle 60.

FIG. 7 illustrates an alternative embodiment of the invention which includes radiopaque strand 85 within tail portion 95. Radiopaque strand 85 may comprise a filamentary strand and Barium Sulfate loaded polymer, Bismuth Trioxide loaded polymer, Platinum, Iridium, Gold, or Tantalum or an alloy thereof.

The occlusion device of the instant invention can be manufactured with relative ease. The filamentary strands of suitable material can be gathered or bunched to form the tail-like structure. The body portion can be constructed of a molded polymeric material, and an end of the tail-like structure incorporated into the mold before forming. The device may be manufactured by inserting or dipping one end of the gathered filamentary strands into polymer in solution and cured and/or allowed to dry. Alternatively, the tail portion may be tied, bonded, or adhered to a metallic body portion.

An improved occlusion device has been disclosed. Although the present invention has been described in accordance with the embodiments shown, one of ordinary skill in the art will readily recognize that there could be variations to the embodiments and those variations would be within the scope of the present invention as defined by the claims.

A wide variety of suitable materials used for filamentary strands and body portions may be interchanged without diverging from the methods or structures of the invention claimed. Similarly, materials conferring radiopacity, thrombogenicity, or other pharmaceutically based therapies may be varied.

## Claims

1. A device (10) for occluding fluid flow through a lumen (60), said device comprising:
a delivery configuration and a treatment configuration.

2. The device according to claim 1 whereby fluid flow in said lumen reconfigures said device from said delivery configuration to said treatment configuration.

3. The device according to claim 1 or 2 wherein said device comprises at least one filamentary strand (16).

4. The device according to claim 3 wherein said device further comprises a body portion (12).

5. The device according to claim 4 wherein said body portion (12) encapsulates a portion of said at least one filamentary strand (16).

6. The device according to claim 3, 4 or 5 wherein the at least one filamentary strand (16) comprises a material selected from the group consisting essentially of silk, cotton, wool, cellulose, nylon, polyethylene, polyethylene terephthalate, polypropylene, polyester, polyurethane, collagen and metals.

7. The device according to claim 4, 5 or claim 6 when dependent of claim 4 or 5 wherein said body portion (12) comprises a material selected from the group consisting essentially of polyurethane, silicone, polyethylene, polyethylene terephthalate, polypropylene, collagen, polytetraflouroethylene, and metals.

8. The device according to any of claims 3 to 7 wherein the at least one filamentary strand (36) is coated with a material (38) which enhances thrombosis.

9. The device according to any of claims 3 to 7 wherein the at least one filamentary strand (36) is impregnated with a material (38) which enhances thrombosis.

10. The device according to claim 8 or 9 wherein the thrombosis enhancing material is selected from the group consisting essentially of collagens, peptides, polymers, thrombins and proteins.

11. The device according to claim 4 or any claim when dependent thereon, wherein said body portion (22) further comprises means (26) for conferring radiopacity.

12. The device according to claim 11 wherein said body portion comprises a material selected from the group consisting of Barium Sulfate, Bismuth Trioxide, Platinum, Iridium, Gold, Tantalum, or an alloy of said materials.

13. The device according to any of claims 3 to 12 wherein said at least one filamentary strand (16) is coated with a therapeutic agent.

14. The device according to any of claims 3 to 13 wherein said at least one filamentary strand (24) comprises means for conferring radiopacity.

15. The device according to claim 14 wherein said at least one filamentary strand comprises a material selected from the group consisting of Barium Sulfate, Bismuth Trioxide, Platinum, Iridium, Gold, Tantalum, or an alloy of said materials.

16. The device according to any preceding claim, said device being adapted to be delivered to the lumen via a microcatheter (50).

17. The device of any preceding claim, said device further comprising a tail portion (14) having a proximal end, a distal end, and a body portion (12) at the distal end;
whereby said proximal end is spaced from said distal end when the device is in the delivery configuration;
and said proximal end is in close proximity to said distal end when the device is in the treatment configuration.

18. A system for occluding fluid through a lumen, said system comprising:
a catheter comprising a fluid medium;
an occlusion device as claimed in any preceding claim, within said fluid.

19. The system according to claim 18 wherein said fluid further comprises a therapeutic agent.

20. The system according to claim 18 wherein said fluid further comprises a diagnostic agent.
